# EUROPÄISCHE PATENTSCHRIFT

(11) **EP 2 682 608 B1**
(45) Veröffentlichungstag und Bekanntmachung des Hinweises auf die Patenterteilung: **16.09.2015**
(21) Anmeldenummer: 13174719.8
(22) Anmeldetag: 02.07.2013
(51) Int. Cl.: F04B 43/12, F04B 53/22

(54) **Multikonnektor und medizinisches Gerät zur extrakorporalen Blutbehandlung mit Multikonnektorerkennung**
Multiconnector and medical device for extracorporeal blood treatment with multiconnector detection
Multiconnecteur et appareil médical pour le traitement extracorporel du sang avec détection de multiconnecteur

(30) Priorität: 03.07.2012 DE 102012105918
(43) Veröffentlichungstag der Anmeldung: 08.01.2014
(73) Patentinhaber: B. Braun Avitum AG, 34212 Melsungen (DE)
(72) Erfinder: Schäfer, Oliver, 36286 Neuenstein (DE); Möller, Dirk, 34326 Altmorschen (DE); Stenzel, Bruno, 26209 Hatten (DE)
(74) Vertreter: Winter, Brandl, Fürniss, Hübner, Röss, Kaiser, Polte - Partnerschaft mbB

(56) Entgegenhaltungen:
- WO-A1-2005/111424
- US-A- 3 963 023
- US-A- 5 752 813
- US-A1- 2002 151 838
- US-A1- 2009 087 326

## Beschreibung

Die Erfindung betrifft einen Multikonnektor zur Anbringung eines Schlauchsegments und zu- und abführenden Leitungen eines extrakorporalen Blutkreislaufs an der Schlauchrollenpumpe eines medizinischen Geräts.

Die Erfindung betrifft ferner ein medizinisches Gerät zur extrakorporalen Blutbehandlung mit Mitteln zur Detektion eines solchen Multikonnektors.

In medizinischen Geräten zur extrakorporalen Blutbehandlung (Dialyse) werden oftmals Schlauchrollenpumpen eingesetzt, welche das entnommene Blut des Patienten zu einem Dialysator und zum Patienten zurückfördern. Solche Schlauchrollenpumpen arbeiten peristaltisch, wobei ein schlaufenförmiges Schlauchsegment an einer entsprechend gebogenen Lauffläche des Pumpengehäuses anliegt. Ein innerhalb der Lauffläche liegender Rotor der Pumpe bewegt sich dann mit seinen Außenkanten entlang des Schlauchsegments, wobei es den Schlauch lokal eindrückt und so mit den elastischen Materialeigenschaften des Schlauchsegments eine Blutförderung durch das Schlauchsegment ermöglicht. Dafür wird das Blut dem Schlauchsegment über einen ersten Anschluss zugeführt und über einen weiteren Anschluss am anderen Ende des Schlauchsegments wieder abgeführt. Das Schlauchsegment bildet so beispielsweise zusammen mit den zu- und abführenden Leitungen und mehreren Luftfängern ein sogenanntes Überleitsystem, mit dem Blut des Patienten zu einem Dialysator und zum Patienten zurückgefördert wird.

Derartige Überleitsysteme werden vorzugsweise nach jeder Behandlung ausgetauscht und nicht wieder für andere Patienten verwendet. Ein benutztes Schlauchsegment muss somit aus der Pumpe entfernt werden, bevor ein neues Überleitsystem in das Gerät eingebracht wird. Um die Handhabung während dieses Ablegens und Aufrüstens des Überleitsystems zu erleichtern, ist es bekannt, an beiden Enden des Schlauchsegments jeweils einen Konnektor vorzusehen, der mit einer zu- bzw. abführenden Leitung verbunden werden kann. Allerdings dienen derartige Konnektoren lediglich als Verbindungsstücke und erfüllen keinerlei weitere Funktionen. Ein Nachteil solcher manueller Systeme mit Konnektoren besteht darin, dass es beim Einlegen in die Pumpe zu Verwechslungen kommen kann. Dieses Problem kann beispielsweise durch eine Farbcodierung und/oder eine geometrische Codierung gelöst werden.

Zur Aufnahme eines Schlauchs innerhalb einer Rollenpumpe ohne Konnektoren beschreibt beispielsweise das Patent US 8,047,819 B2 Haltevorrichtungen, die lösbar am Pumpengehäuse angebracht sind. So können für verschiedene Schlauchgrößen und -arten unterschiedliche Haltevorrichtungen an der Pumpe montiert werden. Eine Haltevorrichtung weist dabei eine Klemmvorrichtung mit wenigstens einer schwenkbaren Klemmbacke auf, die eine halbkreisförmige Ausnehmung aufweist, so dass ein Schlauch in dieser halbkreisförmigen Ausnehmung und einer gegenüber liegenden, ebenfalls halbkreisförmigen Ausnehmung einer anderen Klemmbacke gehalten werden kann. Die Klemmbacken können auch mehrere dieser Ausnehmungen aufweisen, so dass mehrere Schläuche gleichzeitig aufgenommen werden können.

Ferner sind Multikonnektoren bekannt, die beide Anschlüsse für zu- bzw. abführende Leitungen in einem Bauteil vereinigen, das dann in eine Aufnahme des Pumpengehäuses eingebracht werden kann. Über die geometrische Form derartiger Multikonnektoren kann auch ihre Präsenz in der Pumpe detektiert werden, indem beispielsweise ein zylindrischer Abschnitt des Multikonnektors während des Einsetzvorgangs einen Stößel betätigt, dessen axiale Position über eine Lichtschranke abgefragt wird. Gleichzeitig ist dieser Stößel Bestandteil eines im Pumpengehäuse montierten elektromechanischen Aktuators, der den Multikonnektor über einen Linearantrieb auswerfen kann. Ein derartiges System ist bspw. in US 2009/0087326 beschrieben.

Über die Endlagen des Linearantriebs eines solchen Systems können dann auch die Auswurfposition und die Therapie- bzw. Einfädelposition erkannt werden. Diese Positionsabfrage ist jedoch oftmals nicht zufriedenstellend, da lediglich die Therapieposition bzw. Einfädelposition direkt erkannt werden kann. In der Ausfädelposition verfügt das System nur über eine indirekte Detektierung, und auch der Weg von der Therapieposition in die Ausfädelposition kann nur indirekt erkannt werden. Das System kann nur über eine Endlagendetektierung des Linearantriebs die Ausfädelposition detektieren, aber die tatsächliche Position des Multikonnektors ist nicht detektierbar.

Ferner ist es bei solchen Systemen erforderlich, einen Schalter/Knopf an dem medizinischen Gerät zu bedienen bzw. einen Softwarebutton auf einer Benutzeroberfläche zu berühren, um den Ausfädelvorgang zu starten.

Ausgehend vom Stand der Technik ist es die Aufgabe der Erfindung, einen Konnektor zur Anbringung eines Schlauchsegments und zu- und abführenden Leitungen eines extrakorporalen Blutkreislaufs an einer Schlauchrollenpumpe bereitzustellen, dessen aktuelle Position direkt erkannt werden kann.

Aufgabe der Erfindung ist es ferner, ein medizinisches Gerät zur extrakorporalen Blutbehandlung bereitzustellen, an dem die Position eines daran angebrachten Multikonnektors direkt erkannt werden kann.

Erfindungsgemäß wird diese Aufgabe durch einen Multikonnektor gemäß dem unabhängigen Anspruch 1 gelöst. Vorteilhafte Weiterbildungen des Multikonnektors ergeben sich aus den Unteransprüchen 2 bis 9. Die Aufgabe wird ferner durch ein medizinisches Gerät zur extrakorporalen Blutbehandlung gemäß Anspruch 10 gelöst, wobei sich vorteilhafte Ausführungen dieses Geräts aus den Unteransprüchen 11 bis 16 ergeben.

Der erfindungsgemäße Multikonnektor eignet sich zur Anbringung eines Schlauchsegments und zu- und abführenden Leitungen eines extrakorporalen Blutkreislaufs an einer Schlauchrollenpumpe, wobei der Multikonnektor wenigstens einen Grundkörper und zwei Konnektorelemente zur jeweiligen Verbindung des Schlauchsegments mit der zuführenden Leitung und der abführenden Leitung aufweist. Dabei ist der Multikonnektor so ausgebildet, dass er an der Schlauchrollenpumpe in wenigstens zwei Stellungen anbringbar ist, wobei eine erste Stellung eine Therapieposition und eine zweite Stellung eine Ausfädelposition für den extrakorporalen Blutkreislauf darstellt. Die Geometrie und/oder das Material des Multikonnektors sind so ausgestaltet, dass wenigstens diese zwei Stellungen des Multikonnektors direkt durch Detektionsmittel an der Schlauchrollenpumpe detektierbar sind.

Die Multikonnektorerkennung muss somit nicht indirekt beispielsweise über Zustände von Komponenten der Schlauchrollenpumpe bzw. anderen Komponenten eines medizinischen Geräts erfolgen, sondern es kann eine direkte Detektion des Multikonnektors durchgeführt werden. Um eine solche direkte Detektion zu ermöglichen, sind die Geometrie und/oder das Material des Multikonnektors entsprechend ausgestaltet. Die Erfindung nutzt somit nicht nur ohnehin vorhandene Eigenschaften des Multikonnektors und passt die möglichen Detektionsmittel der Schlauchrollenpumpe an diese an, sondern der erfindungsgemäße Multikonnektor ist speziell ausgestaltet, um eine oder mehrere gewünschte Detektionsarten zu ermöglichen.

Gemäß einem ersten Aspekt der Erfindung ist der Grundkörper des Multikonnektors in wenigstens einem Bereich lichtundurchlässig oder lichtzerstreuend, und ist angrenzend an diesen Bereich oder innerhalb dieses Bereichs ein lichtdurchlässiger Ausbruch vorgesehen. Dabei kann der Grundkörper außerhalb des lichtundurchlässigen oder lichtzerstreuenden Bereichs transparent sein, oder der gesamte Grundkörper ist als lichtundurchlässiger oder lichtzerstreuender Bereich ausgeführt. Ist das Material des Grundkörpers transparent, kann für die Konnektorerkennung ein lichtzerstreuender oder lichtundurchlässiger Bereich wie beispielsweise ein Prisma eingesetzt werden. Die gleiche Funktion kann auch mit einer lokalen Bedruckung (Tampondruck) oder einem Aufkleber erreicht werden. Ist das Material des Grundkörpers nicht transparent, genügt gegebenenfalls diese Materialeigenschaft, um die Konnektorerkennung durchzuführen. Das Gleiche gilt für das Material der Konnektor-elemente. In dieser Ausführungsform der Erfindung erfolgt die Multikonnektorerkennung über die Geometrie und die Materialeigenschaften des Multikonnektors, wobei diese Eigenschaften für eine optische Detektion genutzt werden.

Bei einer solchen Ausgestaltung eines Multikonnektors ist es dann beispielsweise möglich, über wenigstens eine Lichtschranke zu ermitteln, in welcher Stellung sich der Multikonnektor gerade befindet, denn das Licht einer Lichtschranke wird entweder unterbrochen, wenn es auf einen lichtundurchlässigen oder lichtzerstreuenden Bereich trifft, oder kann den Grundkörper im Bereich des Ausbruchs durchdringen. Ein wenigstens teilweise transparenter Multikonnektor hat dabei den Vorteil, dass Licht nicht nur durch ihn hindurch geleitet werden kann, um von einer Lichtschranke detektiert zu werden. Vielmehr kann die Position der Sendedioden der Lichtschranke hierdurch auch freier gewählt werden, denn es besteht die Möglichkeit, dass das Licht einer Lichtschranke erst einen transparenten Teilbereich des Multikonnektors durchdringt, bevor es auf einen Ausbruch oder einen lichtundurchlässigen oder lichtzerstreuenden Bereich trifft. Dies erleichtert die Gestaltung der Sensorik am Pumpengehäuse.

Gemäß einem alternativen Aspekt der Erfindung bilden die Geometrie und das Material des Multikonnektors einen Lichtleiter, durch den Licht entlang eines definierten Weges durch den Multikonnektor leitbar ist. Vorzugsweise ist der Lichtleiter dabei so ausgebildet, dass Licht von einem ersten Konnektorelement durch den Grundkörper zu einem zweiten Konnektorelement leitbar ist. Durch eine entsprechende Anordnung von wenigstens einer Sendediode, die Licht in den Multikonnektor einkoppelt, und wenigstens einer Empfangsdiode, die Licht aus dem Multikonnektor empfängt, können so mittels einer Sensorik verschiedene Stellungen des Multikonnektors erkannt werden, indem die Dioden entsprechend in diesen verschiedenen Stellungen angeordnet werden. In dieser Ausführungsform der Erfindung erfolgt die Multikonnektorerkennung somit ebenfalls über die Geometrie und die Materialeigenschaften des Multikonnektors, wobei diese Eigenschaften für eine optische Detektion genutzt werden.

Der Multikonnektor kann dabei insbesondere vom Benutzer manuell in die Ausfädelposition gebracht werden, und sobald diese Ausfädelposition direkt erkannt wurde, kann der Ausfädelvorgang gestartet werden, ohne dass hierfür ein Schalter oder Button betätigt werden muss.

Der Multikonnektor und damit seine beiden Konnektoren können in einer ersten Ausführungsform der Erfindung fest mit dem Schlauchsegment und den zu- und abführenden Leitungen des extrakorporalen Blutkreislaufs verbunden sein. Dabei sind auf einer Seite des Multikonnektors die beiden Enden des Schlauchsegments, welches für die Pumpfunktion in die Schlauchrollenpumpe eingelegt wird, mit den Konnektorelementen verbunden, wodurch sich eine Schlauchschleife ergibt. Auf der anderen Seite des Multikonnektors ist jeweils eine zu- und eine abführende Leitung mit den Konnektorelementen verbunden. Diese Verbindungen der Schläuche mit dem Multikonnektor können durch Verklebung hergestellt werden.

In einer zweiten Ausführungsform der Erfindung weisen die Konnektorelemente des Multikonnektors jeweils wenigstens eine Aufnahme zur lösbaren Verbindung des Schlauchsegments und der zu- und abführenden Leitungen mit dem Multikonnektor auf. Der Multikonnektor bildet dann einen wiederverwendbaren Adapter, der beispielsweise mit einem Standard-Überleitungssystem verbindbar ist, wodurch die vorteilhafte Ausbildung des Multikonnektors auch für ein Standard-Überleitsystem genutzt werden kann. Beispielsweise ist ein Schlauchsegment über einzelne Standard-Konnektorelemente mit den zu- und abführenden Leitungen verbunden, und das Schlauchsegment kann dann für eine lösbare Verbindung in Aufnahmen innerhalb der Konnektorelemente des Multikonnektors eingedrückt werden. Anschließend kann der Multikonnektor zusammen mit dem daran angebrachten Überleitsystem an der Schlauchrollenpumpe angebracht werden.

Diese zweite Ausführungsform des erfindungsgemäßen Multikonnektors als Adapter für beliebig ausgebildete Überleitsysteme hat ferner den Vorteil, dass das Material des Multikonnektors nicht biokompatibel sein muss, da es keinen direkten Kontakt mit dem Blut des Patienten hat.

Ein weiteres Ausführungsbeispiel der Erfindung sieht vor, dass am Grundkörper und/oder den Konnektorelementen wenigstens ein Magnet angebracht ist. Der Magnet kann beispielsweise von dem Kunststoff umspritzt sein, aus dem der Multikonnektor besteht, oder es handelt sich um einen plastizifierten Magneten. Durch eine magnetisch wirkende Sensorik beispielsweise mit Hall-Sensoren an verschiedenen Positionen können dann verschiedene Stellungen des Multikonnektors detektiert werden. In dieser Ausführungsform der Erfindung erfolgt die Multikonnektorerkennung somit wenigstens über die Materialeigenschaften des Multikonnektors, da ein magnetischer Bereich vorhanden sein muss, aber auch die Geometrie des Bauteils ist entscheidend, um die verschiedenen Stellungen des Multikonnektors sicher erkennen zu können. Dazu muss der Magnet in einem bestimmten Bereich angebracht sein, wobei dieser Bereich besonders ausgeformt sein kann, um ihn durch die Sensorik erfassen zu können.

Das gilt ebenso für eine Ausführungsform eines Multikonnektors, bei dem am Grundkörper und/oder den Konnektorelementen wenigstens ein ferromagnetisches Material angebracht ist. Auch das ferromagnetische Material kann beispielsweise von Kunststoff umspritzt sein. Hierdurch ist es möglich, verschiedene Stellungen des Multikonnektors durch eine induktiv wirkende Sensorik zu erfassen, wobei die Detektion anhand des Geometrie und der Materialeigenschaften erfolgt.

In einem bevorzugten Ausführungsbeispiel der Erfindung sind die Konnektorelemente an zwei gegenüber liegenden Seiten des Grundkörpers angebracht, wobei die Außenkontur eines ersten Konnektorelements wenigstens einen zylindrischen Bereich mit einer Zylinderachse aufweist. Abseits des ersten Konnektorelements sind dann Mittel zum Verrasten des Multikonnektors mit einem Pumpengehäuse der Schlauchrollenpumpe vorgesehen, wobei die mit diesen Mitteln verrastete Stellung des Multikonnektors die Therapieposition darstellt. Durch diesen Aufbau des Multikonnektors ist es möglich, das erste Konnektorelement in eine Aufnahme an dem Pumpengehäuse einer Schlauchrollenpumpe einzubringen, wobei sich durch den zylindrischen Bereich des Konnektorelements ein Drehlager bilden lässt, so dass der Multikonnektor um die Zylinderachse des ersten Konnektorelements schwenkbar ist. Dies kann beim Vorgang des Einsetzens und Herausnehmens des Multikonnektors vorteilhaft genutzt werden. Die Ausfädelposition kann dann dadurch erreicht werden, dass der Multikonnektor um das Drehlager vom Pumpengehäuse weg in eine definierte Endlage geschwenkt wird. In dieser Endlage ist der Multikonnektor vorzugsweise ebenfalls arretierbar. Dies kann beispielsweise durch entsprechende Mittel im Bereich des Drehlagers bzw. des ersten Konnektorelements erfolgen, wobei sich Rastmechanismen als zweckmäßig erwiesen haben.

Von der Erfindung umfasst ist ferner ein medizinisches Gerät zur extrakorporalen Blutbehandlung, umfassend wenigstens eine Schlauchrollenpumpe mit einem Pumpengehäuse, das eine gebogene Lauffläche und einen innerhalb der Lauffläche drehenden Rotor aufweist, wobei ein Schlauchsegment eines extrakorporalen Blutkreislaufs zwischen die Lauffläche und den Rotor einbringbar ist. Dem Schlauchsegment ist Blut über eine zuführende Leitung zuführbar, während Blut von dem Schlauchsegment über eine abführende Leitung abführbar ist. Am Pumpengehäuse ist ein erfindungsgemäßer Multikonnektor in wenigstens zwei Stellungen anbringbar, wobei eine erste Stellung eine Therapieposition und eine zweite Stellung eine Ausfädelposition darstellt. Das medizinische Gerät weist ferner Detektionsmittel zur direkten Detektion wenigstens dieser zwei Stellungen des Multikonnektors auf, wobei die Detektion mittels der Geometrie und des Materials des Multikonnektors durchführbar ist.

Zur Anbringung des Multikonnektors am Pumpengehäuse kann an diesem eine Aufnahme vorgesehen sein, in welche ein zylindrischer Bereich des ersten Konnektorelements einbringbar ist, wodurch ein Drehlager gebildet wird, durch das der Multikonnektor um die Zylinderachse des ersten Konnektorelements schwenkbar ist. Am Pumpengehäuse sind ferner Mittel zum Verrasten eines Bereichs des Multikonnektors am Pumpengehäuse vorgesehen, der abseits des ersten Konnektorelements liegt. Ein derartig verrasteter Multikonnektor befindet sich in der Therapiestellung bzw. Einfädelposition.

Um wenigstens zwei Stellungen eines Multikonnektors erfassen zu können, dessen Grundkörper in wenigstens einem Bereich lichtundurchlässig oder lichtzerstreuend ist, und bei dem angrenzend an diesen Bereich oder innerhalb dieses Bereichs ist ein lichtdurchlässiger Ausbruch vorgesehen ist, umfassen die Detektionsmittel beispielsweise eine Lichtschranke. Mit dieser Lichtschranke ist Licht durch den Grundkörper des Multikonnektors sendbar, wobei die Lichtschranke so positioniert ist, dass der lichtdurchlässige Ausbruch des Multikonnektors in einer der Stellung des Multikonnektors von der Lichtschranke durchleuchtbar ist, während dieser lichtdurchlässige Ausbruch in der zweiten Stellung des Multikonnektors nicht von der Lichtschranke durchleuchtbar ist. Die zugehörige Sensorik kann diese beiden Zustände auswerten, woraus auf die jeweilige Stellung des Multikonnektors geschlossen werden kann.

Um wenigstens zwei Stellungen eines Multikonnektors erfassen zu können, bei dem die Geometrie und das Material einen Lichtleiter bilden, durch den Licht entlang eines definierten Weges durch den Multikonnektor leitbar ist, umfassen die Detektionsmittel wenigstens eine Sendediode, mit der Licht in den Multikonnektor einkoppelbar ist. Ferner sind wenigstens zwei Empfangsdioden vorgesehen, wobei eine erste Empfangsdiode so positioniert ist, dass Licht in einer ersten Stellung des Multikonnektors von der Sendediode zu dieser ersten Empfangsdiode leitbar ist, während eine zweite Empfangsdiode so positioniert ist, dass Licht in einer zweiten Stellung des Multikonnektors von der Sendediode zu dieser zweiten Empfangsdiode leitbar ist. Hieraus kann auf die jeweilige Stellung des Multikonnektors geschlossen werden.

Vorzugsweise ist die Sendediode dabei im Bereich der Aufnahme angebracht, während eine erste Empfangsdiode in einem Bereich angebracht ist, in dem das zweite Konnektorelement in der Therapieposition positioniert ist, und eine zweite Empfangsdiode ist in einem Bereich angebracht, in dem das zweite Konnektorelement in der Ausfädelposition positioniert ist.

Darüber hinaus können die Detektionsmittel alternativ oder ergänzend wenigstens einen Hall-Sensor und/oder einen induktiv wirkenden Näherungssensor umfassen, mit dem mittels des Materials in Kombination mit der Geometrie des Multikonnektors jeweils wenigstens zwei Stellungen des Multikonnektors detektierbar sind. Diese Detektionsmittel eignen sich insbesondere zur Erkennung von Multikonnektoren mit Magneten und/oder ferromagnetischen Materialien. Kapazitiv wirkende Sensoriken wie kapazitive Näherungssensoren können dagegen verwendet werden, um die Materialeigenschaften und damit die Permittivitätszahl für eine Detektion verschiedener Stellungen des Multikonnektors zu nutzen. Dabei werden mehrere kapazitive Näherungssensoren so am Pumpengehäuse angebracht, dass sie verschiedene Stellungen kapazitiv erfassen können.

Ferner können die Detektionsmittel wenigstens einen Distanzsensor umfassen, mit dem ein veränderlicher Abstand zwischen dem Distanzsensor und dem Multikonnektor in den wenigstens zwei Stellungen des Multikonnektors messbar ist. Hierbei kann die Detektion über die geometrischen, materiellen und optischen Eigenschaften des Multikonnektors, da der Multikonnektor entsprechend ausgeformt sein kann, um die gewünschte Distanzmessung durchführen zu können. Die Abstandsmessung kann beispielsweise über das Prinzip der optischen Triangulation zur berührungslosen Positionsbestimmung erfolgen. Ein von einem Sensor ausgehender Laserstrahl produziert dabei auf der Oberfläche des Multikonnektors einen Lichtpunkt. Dieser Lichtpunkt wird über eine hochwertige Optik auf einen positionsempfindlichen Detektor projiziert. Die Technologie beruht auf der Messung der Zeit zwischen Aussendung und Empfang von Laserlichtimpulsen und lässt bei einer 12-Bit-Auswertung eine präzise Entfernungsmessung zu. Dabei kann insbesondere die Oberfläche des Multikonnektors ausgebildet sein, um den Lichtpunkt geeignet zu projizieren.

In einem weiteren Ausführungsbeispiel der Erfindung erfolgt eine Multikonnektorerkennung über eine oder mehrere Betätigungsgeometrien, die am Multikonnektor ausgeformt sind. Diese können beispielsweise erhaben oder vertieft ausgeführt sein und eine mechanisch wirkende Sensorik am Pumpengehäuse betätigen. Bei der mechanisch wirkenden Sensorik handelt es sich beispielsweise um einen Mikroschalter, der mit einer bestimmten Geometrie am Multikonnektor in Kontakt kommen kann, woraus auf die Stellung des Multikonnektors geschlossen werden kann. Hierbei erfolgt die Multikonnektorerkennung somit über die Geometrie des Multikonnektors.

Die genannten Detektionsarten und die zugehörige Ausbildung eines Multikonnektors können einzeln oder auch in Kombination eingesetzt werden, wobei eine Kombination die Genauigkeit der Multikonnektorerkennung verbessern könnte. Eine Kombination mehrerer Detektionsarten könnte jedoch auch genutzt vorteilhaft sein, wenn eine erste Stellung des Multikonnektors beispielsweise besser durch eine erste Detektionsart erkannt werden kann, während eine andere Detektionsart für eine zweite Stellung günstiger ist. Gegebenenfalls ließe sich hierdurch auch die Konstruktion der Pumpe vereinfachen, da anzubringende Sensoriken nicht die Geometrie der Pumpe bzw. des Pumpengehäuses vorgeben würden, sondern die Sensoriken könnten in Abhängigkeit von einem vorteilhaften Pumpendesign gewählt werden. Insbesondere optische Detektionsarten mit Sende- und Empfangsdioden könnten geeignet kombiniert werden.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Darstellung eines medizinischen Geräts zur extrakorporalen Blutbehandlung mit einer Blutpumpe;
- Fig. 2: eine schematische Darstellung einer Schlauchrollenpumpe mit eingelegtem Schlauchstück und einem Ausführungsbeispiel eines Multikonnektors gemäß einer ersten Ausführungsform;
- Fig. 3a: eine erste schematische Seitenansicht einer Schlauchrollenpumpe nach Fig. 2;
- Fig. 3b: eine schematische Seitenansicht gemäß Fig. 3a mit ausgeschwenktem Multikonnektor;
- Fig. 4a: eine vergrößerte schematische Seitenansicht einer Schlauchrollenpumpe nach Fig. 2;
- Fig. 4b: eine schematische Seitenansicht gemäß Fig. 4a mit ausgeschwenktem Multikonnektor.
- Fig. 5a: eine schematische Seitenansicht einer Schlauchrollenpumpe mit geometrischer Codierung am Multikonnektor;
- Fig. 5b: eine schematische Seitenansicht gemäß Fig. 5a mit ausgeschwenktem Multikonnektor;
- Fig. 6a: eine schematische Seitenansicht einer Schlauchrollenpumpe lichtleitendem Multikonnektor;
- Fig. 6b: eine schematische Seitenansicht gemäß Fig. 6a mit ausgeschwenktem Multikonnektor;
- Fig. 7: eine schematische Darstellung einer Schlauchrollenpumpe mit eingelegtem Schlauchstück und einem als Adapter ausgeführten Multikonnektor gemäß einer zweiten Ausführungsform; und
- Fig. 8: eine schematische Seitenansicht einer Schlauchrollenpumpe nach Fig. 7.

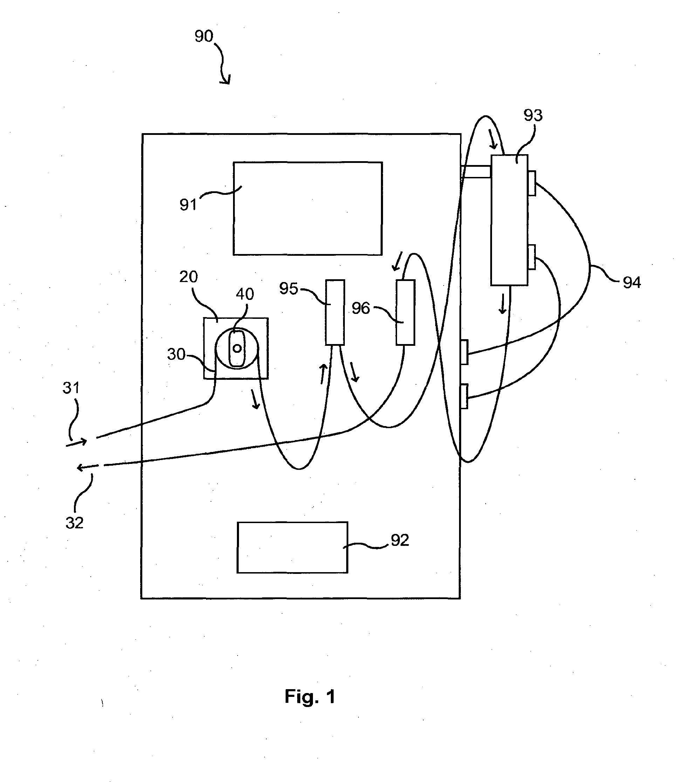

Fig. 1 zeigt eine schematische Darstellung der wesentlichen Grundkomponenten eines medizinischen Geräts bzw. Dialysegeräts 90 zur extrakorporalen Blutbehandlung mit einer Blutpumpe, wobei es sich bei der Blutpumpe um eine Schlauchrollenpumpe handelt. Die Schlauchrollenpumpe weist dabei ein Pumpengehäuse 20 auf, das typischerweise an der Frontseite des Dialysegeräts 90 angebracht ist.

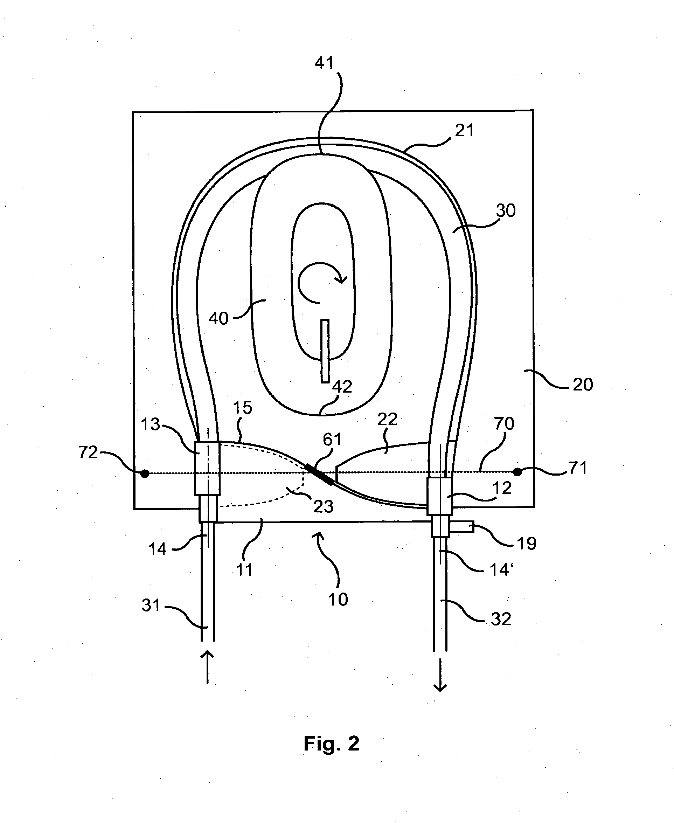

Dieser Schlauchrollenpumpe wird arterielles Blut 31 eines Patienten zugeführt und durch den extrakorporalen Blutkreislauf gefördert. Anschließend wird das Blut als venöses Blut 32 wieder zum Patienten zurückgeführt. Dabei wird das Blut mittels der Pumpe durch ein Überleitsystem gefördert, das an mehrere Komponenten des Dialysegeräts angeschlossen ist, wobei ein Schlauchsegment 30 des Überleitsystems in die Blutpumpe eingelegt ist und ein Rotor 40 das Blut peristaltisch durch dieses Schlauchsegment 30 fördert, wie es einer vergrößerten Ansicht der Fig. 2 zu entnehmen ist.

Nach Durchlaufen der Blutpumpe gelangt das Blut zum Dialysator 93, nachdem es vorzugsweise zuvor einen arteriellen Luftfänger 95 durchlaufen hat. Im Dialysator 93 wird das Blut durch Stoffaustausch mit einem Dialysat 94 gereinigt, welches dem Dialysator 93 zu- und abgeführt wird. Nach Durchlaufen des Dialysators 93 gelangt das Blut zu einem venösen Luftfänger 96 und wird dann dem Patienten zugeführt. Dieser Kreislauf des Bluts des Patienten ist in Fig. 1 durch Pfeile gekennzeichnet.

Die Einstellung von Parametern der Dialyse und die Überwachung der Therapie können über eine Anzeige-/Eingabeeinheit 91 erfolgen, die vorzugsweise als Touchscreen ausgebildet ist. Ferner weist das Dialysegerät 90 eine Steuer-/Regeleinheit 92 auf.

Fig. 2 stellt eine schematische Darstellung einer Schlauchrollenpumpe mit eingelegtem Schlauchsegment 30 und einem ersten Ausführungsbeispiel eines Multikonnektors 10 dar, anhand der die erfindungsgemäße Multikonnektorerkennung erläutert werden soll. Die Schlauchrollenpumpe weist das Pumpengehäuse 20 auf, das für den Bediener des Geräts leicht zugänglich, wobei es mit einem (in Fig. 2 nicht dargestellten) Deckel 50 abdeckbar ist, der über ein Scharnier zum Beispiel nach oben oder zur Seite schwenkbar ist, um Zugriff auf die Pumpe zu erhalten.

In dem Pumpengehäuse 20 ist durch eine Vertiefung im Gehäuse eine kurvenförmige Lauffläche 21 ausgebildet, in welche das Schlauchsegment 30 schlaufenförmig eingelegt werden kann, so dass seine beiden Schlauchenden aus dem Gehäuse 20 herausragen. Dabei kann die Vertiefung mit einer Seitenfläche in dem Pumpengehäuse 20 ausgebildet sein, die im Wesentlichen gleichmäßig senkrecht zur Frontseite des Geräts verläuft, oder die Lauffläche 21 ist ungleichmäßig durch eine Seitenfläche der Vertiefung ausgeformt, die konkav oder sogar in sich verdreht ausgeformt ist.

Innerhalb der Lauffläche 21 ist ein Rotor 40 angebracht, der einen elliptischen Umfang hat, so dass er das Schlauchsegment 30 bei der Rotation an seinen Hauptscheiteln 41, 42 punktuell zusammendrücken kann. Durch die Drehung des Rotors 40 im Uhrzeigersinn bewegt sich der Bereich eines zusammengedrückten Schlauchsegments 30 ebenfalls im Uhrzeigersinn, bis sich der zugehörige Hauptscheitel 41 wieder vom Schlauchsegment 30 löst. In der Zeit hat der gegenüber liegende Hauptscheitel 42 jedoch bereits wieder Kontakt zu dem Schlauchsegment 30 aufgenommen, so dass Blut jeweils in dem Bereich des Schlauchsegments 30, vor dem es von dem Rotor 40 zusammendrückt wird, peristaltisch vom Pumpeneingang zum Pumpenausgang gefördert wird. Der Rotor kann neben einem elliptischen Umfang jedoch auch jegliche andere geeignete Form haben.

An den beiden Enden des schlaufenförmigen Schlauchsegments 30 ist der erfindungsgemäße Multikonnektor 10 angebracht, welcher eine Verbindung zu einer zuführenden Leitung 31 und einer abführenden Leitung 32 herstellt, durch welche somit Blut zu- und abgeführt wird. Diese Leitungen 31, 32 sind Teil des extrakorporalen Blutkreislaufs und sind mit verschiedenen Komponenten wie Luftfängern und dem Dialysator verbunden.

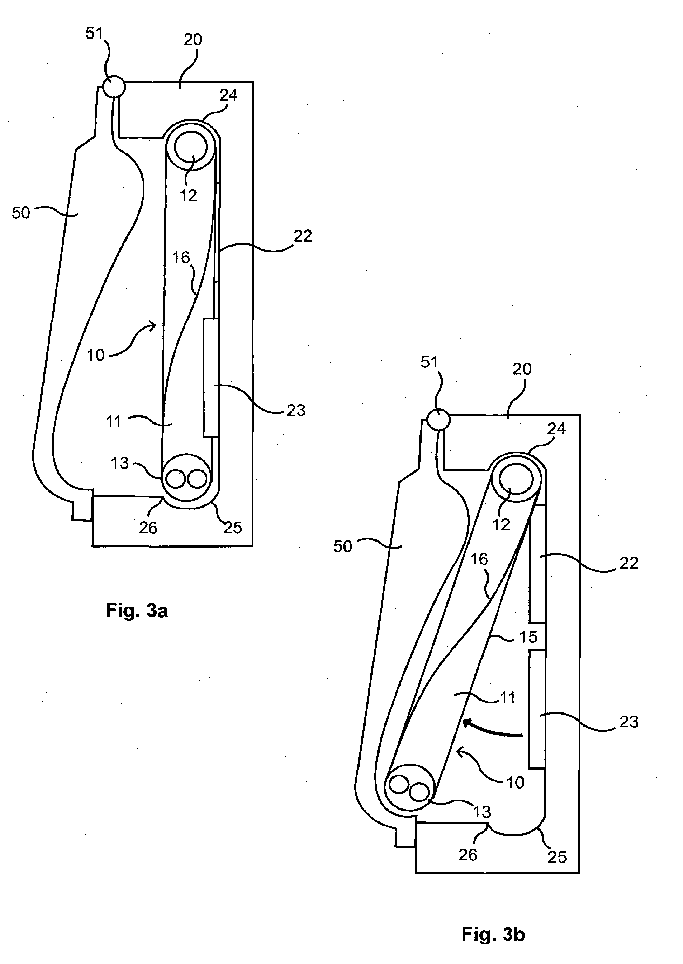

Der Multikonnektor 10 umfasst einen Grundkörper 11, der zwei gegenüber liegende Konnektorelemente 12 und 13 miteinander verbindet, die auch als Konnektoren bezeichnet werden können. Wenigstens das Konnektorelement 12 ist zylinderförmig bzw. hohlzylinderförmig ausgeführt oder weist eine nach außen gewölbte und gleichmäßig gekrümmte Außenflächeauf. Hierdurch kann dieses Konnektorelement 12 in eine entsprechend ausgeformte, insbesondere nach innen gewölbte und gleichmäßig gekrümmte, Aufnahme 24 (siehe Fig. 3a) im Pumpengehäuse 20 werkzeuglos eingebracht bzw. eingelegt werden, wodurch sich ein Drehlager bzw. Gleitlager ergibt, um welches der Multikonnektor 10 bei der Montage und Demontage schwenkbar ist. Das zweite Konnektorelement 13 ist vorzugsweise ebenfalls zylinderförmig bzw. hohlzylinderförmig ausgeformt, wobei es sich auch um einen Zylinder mit abgestuften Außenflächen bzw. Rastflächen handeln kann. Die Zylinderachsen 14 und 14' der beiden Konnektorelemente 12, 13 verlaufen dabei parallel zueinander und quer zur Längserstreckung des Grundkörpers 11 bzw. des Multikonnektors 10, so dass die beiden Leitungen 31, 32 ebenfalls parallel in die Pumpe bzw. aus der Pumpe geführt werden. Die Zylinderachsen und damit die Konnektoren können jedoch auch anders am Grundkörper 11 angebracht sein, wenn beispielsweise eine Zu- und Abführung von Blut in andere Richtungen vorteilhaft ist.

Der Multikonnektor 10 ist somit in Fig. 2 um den Drehpunkt am Konnektorelement 12 aus der Zeichenebene heraus schwenkbar. In der eingeschwenkten Stellung sind Mittel vorgesehen, mit denen der Multikonnektor 10 am Pumpengehäuse 20 verrastbar ist. Beispielsweise ist das zweite Konnektorelement 13 dazu so ausgeformt, dass es in eine Aufnahme 25 im Pumpengehäuse 20 einrastet, wenn es gegen das Pumpengehäuse 20 gedrückt wird. Es kann aber durch leichtes Ziehen auch wieder aus dieser Aufnahme 25 gelöst werden, wenn der Multikonnektor 10 aus der Zeichenebene der Fig. 2 herausgeschwenkt wird. Der Grundkörper 11 des Multikonnektors 10 ist daher vorzugsweise in sich leicht elastisch ausgebildet, so dass er ein Einrasten bzw. einen Formschluss in einer Aufnahme 25 ermöglicht. Er kann jedoch auch relativ steif ausgeführt sein, wobei dann die Aufnahme 25 im Pumpengehäuse 20 elastisch ausgeführt wäre. Bei der Materialauswahl ist ferner zu berücksichtigen, dass zumindest die Konnektoren 12 und 13 biokompatibel sind, da sie direkt mit dem Blut des Patienten in Kontakt kommen.

In der in Fig. 2 dargestellten Ansicht ist wenigstens eine obere Seitenfläche 15 des Grundkörpers 11 S-förmig oder als anders ausgeformte geometrische Codierung ausgeführt. Hierdurch ist es möglich, dass sich der Multikonnektor 10 gegen eine Bewegung entlang der Zylinderachsen 14, 14' am Pumpengehäuse 20 abstützt. Dazu ist am Pumpengehäuse 20 beispielsweise ein entsprechend ausgeformtes Stützelement 22 vorgesehen, das als Erhöhung am Pumpengehäuse 20 ausgeformt ist. An diesem Stützelement 22 liegt die Seitenfläche 15 des Grundkörpers 11 an. Daher kann der Multikonnektor 10 hier im Bereich des Pumpengehäuses 20 nicht in die Pumpe hineingedrückt werden.

Weitere Flächen des Grundkörpers 11 können ebenfalls gebogen oder sogar in sich verdreht ausgebildet sein, wodurch beispielsweise an der rechten Seite des Grundkörpers 11 eine Grifffläche ausgebildet werden kann. An dieser kann der Multikonnektor 10 von einem Bediener gegriffen werden, um ihn dann erst in die obere Aufnahme einzulegen und dann in die Zeichenebene hineinzuschwenken und in der unteren Aufnahme zu verrasten. Um diesen Vorgang näher zu erläutern, zeigen die Figuren 3a und 3b eine erste Seitenansicht des Multikonnektors 10, wobei diese Ansicht in Fig. 2 einer Sicht von unten entspricht.

Dabei zeigt Fig. 3a einen eingelegten Multikonnektor 10, dessen oberes Konnektorelement 12 in eine halbkreisförmige Aufnahme 24 im Pumpengehäuse 20 eingebracht ist. Durch die zylindrische Form des Konnektorelements 12 und der Aufnahme 24 ergibt sich an dieser Stelle ein Gleitlager mit einem Drehpunkt bzw. Drehachse, um den der Multikonnektor 10 schwenkbar ist. Dabei ist das untere Konnektorelement 13 in eine Aufnahme 258 eingerastet. Dies kann beispielsweise durch eine halbkreisförmige Aufnahme 25 erreicht werden, die eine als Rastkante fungierende Kante 26 (linke Kante der Aufnahme 25 in Fig. 3a und 3b) aufweist, die in den Schwenkbereich des Multikonnektors 10 um die Drehachse 14 hineinragt, über die das Konnektorelement 13 erst gedrückt werden muss, um in die Aufnahme 25 zu gelangen. Hierbei handelt es sich um eine erste definierte Stellung des Multikonnektors 10, die als Therapieposition oder Einfädelposition bezeichnet wird. In dieser Stellung wird das Schlauchsegment 30 für die Therapie in die Lauffläche 21 im Pumpengehäuse 20 eingefädelt.

In dieser Ansicht ist auch erkennbar, dass das untere Konnektorelement 13 zwei Öffnungen zum Anschluss von Leitungen hat, die auf der anderen Seite in eine gemeinsame Röhre münden, die dann an das Schlauchsegment 30 in der Pumpe angeschlossen werden kann. So können verschiedene Leitungen angeschlossen werden. Beispielsweise kann eine Öffnung für die Blutzufuhr und ein anderer Anschluss für eine Druckmessung verwendet werden. Ferner kann am oberen Konnektorelement 12 ein weiteres Anschlussrohr 19 vorgesehen sein, das jedoch lediglich in der Figur 1 dargestellt ist und senkrecht zur Zylinderachse 14 verläuft. An diesen Zusatzanschluss 19 kann ebenfalls eine weitere Leitung angeschlossen werden, wobei es sich beispielsweise um eine Heparinzufuhr handeln kann.

Darüber hinaus ist das Stützelement 23 erkennbar, durch welches der Multikonnektor 10 nicht aus der Pumpe herausgezogen werden kann, wobei das andere Stützelement 22 hinter dem Multikonnektor 10 angedeutet ist. Das Pumpengehäuse 20 wird ferner von einem Klappdeckel 50 abgedeckt, der um ein oberes Scharnier 51 schwenkbar ist. Gegen diesen Deckel 50 kann der Multikonnektor 10 bei der Demontage gezogen werden, wie sie in Fig. 3b dargestellt ist. Bei dieser Stellung handelt es sich um eine definierte zweite Stellung des Multikonnektors 10, die als Ausfädelposition bezeichnet wird. In diese Stellung wird der Multikonnektor 10 insbesondere nach der Therapie gebracht, um das Schlauchsegment 30 aus der Lauffläche 21 des Pumpengehäuses 20 zu entnehmen.

Dazu kann der Multikonnektor 10 an seinem Grundkörper 11 gefasst und um den Drehpunkt in der oberen Aufnahme 24 geschwenkt werden, wie es durch den Pfeil dargestellt ist. Hierzu kann eine weitere S-förmige Fläche 16 des Grundkörpers 11 so ausgeformt sein, dass der Bediener den Multikonnektor 10 an dieser Fläche gut greifen kann. Dann muss die Verrastung der unteren Aufnahme 25 überwunden werden, indem das untere Konnektorelement 13 über die Rastkante 26 gezogen wird. Der geschlossene Deckel 50 der Pumpe dient dabei als Gegenlager und gleichzeitig als Endanschlag für den Multikonnektor 10 in der zweiten Endlage.

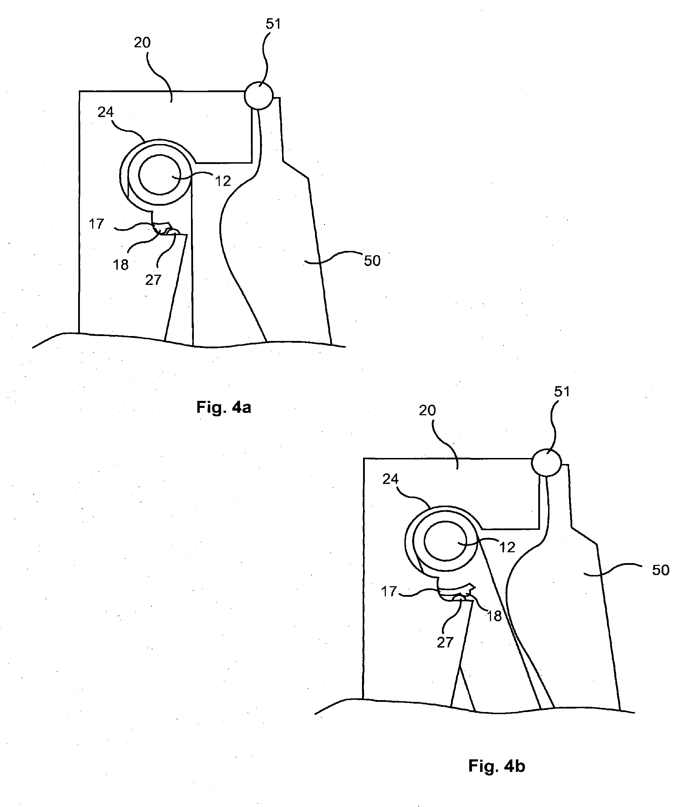

Damit der Multikonnektor 10 in der Ausfädelposition gehalten werden kann, ist beispielsweise am oberen Drehpunkt ein Federmechanismus vorgesehen. Die Funktionsweise dieses Federmechanismus ist den Figuren 4a und 4b zu entnehmen, die eine Sicht auf den Multikonnektor der Fig. 2 von oben zeigen, wobei jedoch lediglich ein vergrößerter Ausschnitt des Drehpunkts dargestellt ist. In der Fig. 4a ist der Multikonnektor 10 erneut in der Therapieposition gezeigt, wobei auf dieser Seite des Grundkörpers 11 ein bogenförmiges Federelement 17 an dem Grundkörper 11 angebracht ist. Dieses Federelement 17 verläuft im Wesentlichen entlang der Außenkontur des zylinderförmigen Konnektorelements 12 und weist ein Rastelement in Form einer Erhöhung 18 auf. Diese Erhöhung 18 stößt in dieser Stellung des Multikonnektors 10 an eine Rastnase 27 an, die an dem Pumpengehäuse 20 ausgeformt ist. Der Multikonnektor 10 ist somit in der Therapieposition am oberen und am unteren Konnektorelement mit dem Pumpengehäuse 20 verrastet.

Beim Ausschwenken des Multikonnektors 10 aus der Therapiestellung in die Ausfädelposition muss dieser nicht nur aus der unteren, sondern auch aus der oberen Verrastung gelöst werden. Dies ist in Fig. 4b dargestellt, wobei ersichtlich ist, dass das obere Konnektorelement 12 beim Schwenken um den Drehpunkt mit dem Rastelement 18 über die Rastnase 27 am Pumpengehäuse 20 gezogen wurde. In dieser Stellung kann der Multikonnektor 10 gehalten und somit arretiert werden, da das Rastelement 18 nun von der anderen Seite an der Rastnase 27 anliegt und alleine durch das Gewicht des Multikonnektors 10 nicht wieder zurückrutscht bzw. zurückschwenkt. Dabei dient der Pumpendeckel 50 vorzugsweise als Gegenlager.

Die Verrastungsvorgänge werden vorzugsweise dadurch erleichtert, dass der Grundkörper 11 durch seine Geometrie aus mehreren gebogenen Flächen elastisch ausgebildet ist. Als Material für den Grundkörper 11 und/oder die Konnektoren 12, 13 wird daher vorzugsweise ein Material mit den entsprechenden elastischen Eigenschaften eingesetzt.

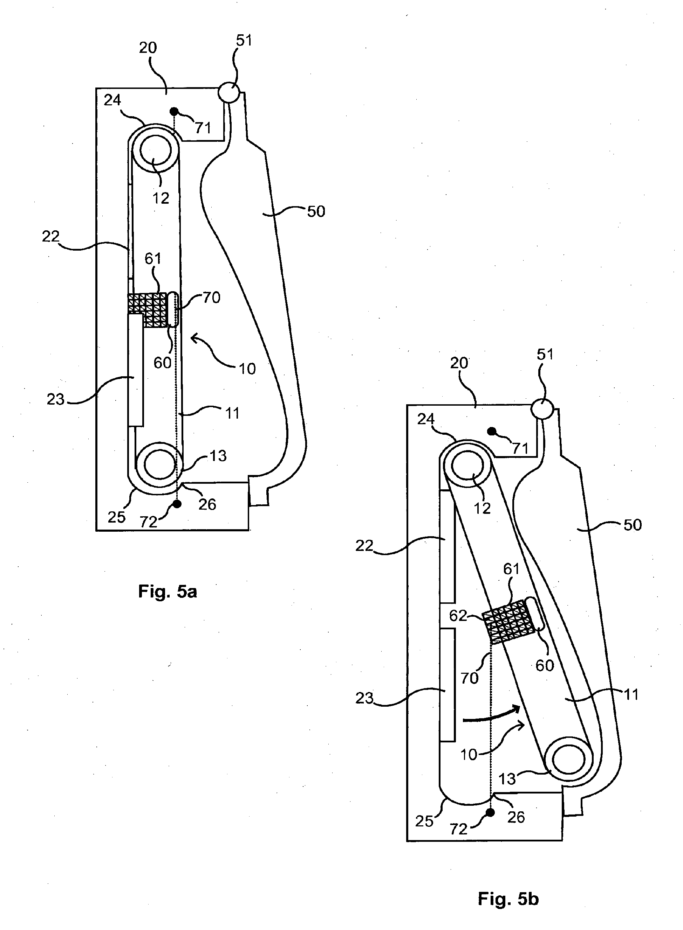

Im ersten Ausführungsbeispiel der Erfindung ist der Multikonnektor 10 so ausgeformt, dass er im Grundkörper 11 einen Ausbruch 60 aufweist, wie er den Figuren 5a und 5b zu entnehmen ist. Der Ausbruch 60 hat beispielsweise die Form eines Langlochs und ist etwa in der Mitte zwischen den beiden Konnektoren 12, 13 angeordnet. Angrenzend an diesen Ausbruch 60 hat ein Bereich des Grundkörpers 11 eine lichtzerstreuende Oberflächenstruktur 61, die beispielsweise durch eine Riffelung und/oder mehrere Prismen erreicht werden kann. Diese Oberflächenstruktur 61 ist in Fig. 2 auf der S-förmigen Seitenfläche 15 dargestellt. Außerhalb dieses Bereiches 61 ist der Grundkörper 11 dabei transparent und somit lichtdurchlässig.

Zur Detektierung der Stellung des Multikonnektors 10 am Pumpengehäuse 20 ist bei einem solchen Multikonnektor 10 wenigstens eine Lichtschranke 70 vorgesehen, die im Infrarot-Bereich arbeitet. Dabei senden mehrere Sendedioden auf unterschiedlichen Frequenzen infrarotes Licht durch den über sein Material und seine Geometrie bestimmten Konnektor 10, wobei das Licht in den unterschiedlichen Stellungen des Multikonnektors durch diesen hindurchgeleitet werden kann oder nicht. Um dies näher zu erläutern, ist in der Fig. 5a gezeigt, an welcher Stelle das Licht einer Lichtschranke 70 den Grundkörper 11 des Multikonnektors im Bereich des Ausbruchs 60 durchdringt. Durch die S-förmige Fläche 15, auf welcher der Ausbruch 60 und die lichtzerstreuende Oberflächenstruktur 61 angebracht sind, kann das infrarote Licht der Sendedioden 71, 72 von zwei Seiten durch diese Seitenfläche dringen, wie es auch in Fig. 2 gezeigt ist. Diese Therapieposition kann somit dadurch detektiert werden, dass die Lichtschranke 70 nicht unterbrochen wird.

Wird der Multikonnektor 10 nun wie in Fig. 5b in die Ausfädelposition bewegt, gerät die lichtzerstreuende Oberflächenstruktur 61 in die Lichtschranke 70, so dass der Lichtstrahl der Lichtschranke 70 unterbrochen wird und die zugehörige Sensorik erkennt, dass sich der Multikonnektor 10 in einer Stellung befindet, in welcher sich die Oberflächenstruktur 61 im Bereich der Lichtschranke 70 befindet, d.h. in der Ausfädelposition. Dazu weist der Grundkörper 11 im dargestellten Ausführungsbeispiel im Bereich der Oberflächenstruktur 61 eine Ausbuchtung bzw. Vorsprung 62 auf, auf welcher die Oberfläche ebenfalls lichtundurchlässig bzw. lichtzerstreuend ausgebildet ist. Diese Geometrie kann erforderlich sein, damit das Licht der Lichtschranke in der Ausfädelposition nicht auf einen lichtdurchlässigen Bereich fällt, sondern auf irgendeine Art vom Grundkörper 11 blockiert wird. Die Ausbuchtung 62 stellt somit eine Verlängerung der lichtstreuenden Oberfläche 61 dar.

Um weitere Stellungen beispielsweise zwischen den beiden Endlagen detektieren zu können, können auch mehrere Lichtschranken mit Sendedioden vorgesehen sein, die auf unterschiedlichen Frequenzen senden. Ferner kann die Sensorik auch so gestaltet bzw. die Lichtschranke 70 so positioniert sein, dass die Therapieposition durch eine unterbrochene Lichtschranke 70 gekennzeichnet ist, während die Ausfädelposition durch eine nicht unterbrochene Lichtschranke 70 gekennzeichnet ist. Der Multikonnektor 10 kann dazu auch weitere Ausbrüche und/oder lichtundurchlässige bzw. lichtzerstreuende Flächen aufweisen. Lichtundurchlässige Bereiche können beispielsweise durch lichtundurchlässiges Material, eine Einfärbung des Materials des Grundkörpers 11 und/oder eine lichtundurchlässige Beschichtung erreicht werden.

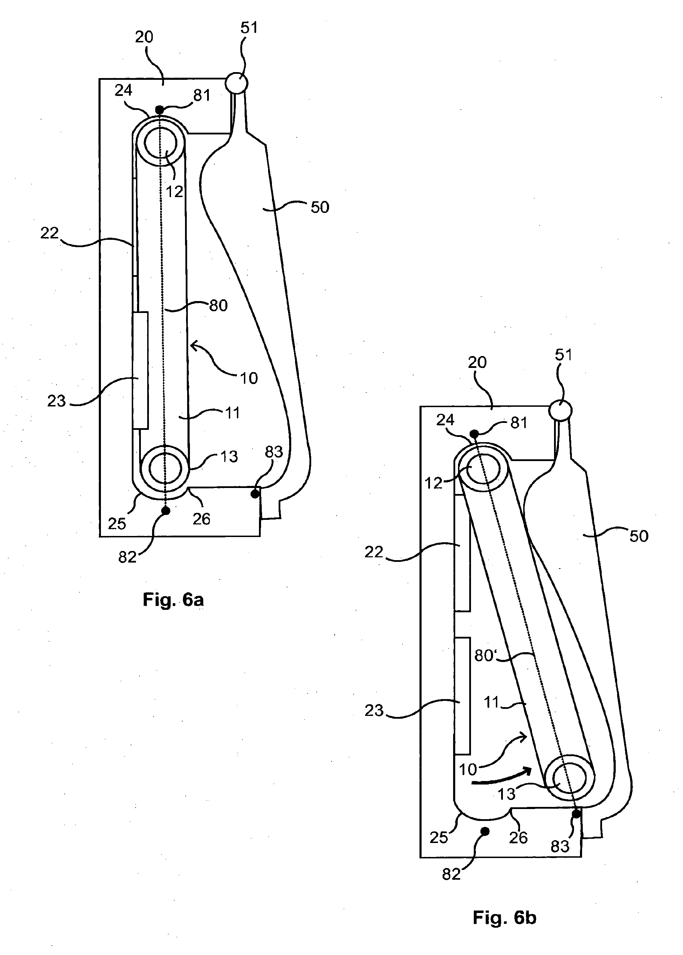

Ergänzend oder alternativ sind die Geometrie und das Material des Grundkörpers 11 so gewählt, dass der Multikonnektor 10 als Lichtleiter genutzt werden kann, durch den Licht entlang eines definierten Weges leitbar ist. Vorzugsweise ist dieser Lichtleiter so ausgeführt, dass Licht von einem Konnektorelement zum anderen leitbar ist. Wie aus den Figuren 6a und 6b ersichtlich, ist beispielsweise im Bereich der oberen Aufnahme 24 und damit beim oberen Konnektorelement 12 eine Sendediode 81 positioniert, die dort Licht in den Multikonnektor 10 einkoppelt. Das Licht gelangt über einen ersten Lichtleiterweg 80 zum unteren Konnektorelement 13 und tritt dann aus dem Multikonnektor 10 aus zu einer Empfängerdiode 82 im Bereich der unteren Aufnahme 25. Diese Therapieposition kann somit durch eine zugehörige Sensorik detektiert werden.

Wird der Multikonnektor 10 in die Ausfädelposition bewegt, wie es Fig. 6b zeigt, wird das Licht durch den zweiten Lichtleiterweg 80' hingegen zu einer zweiten Empfängerdiode 83 im Bereich des Pumpengehäuses 20 geleitet, in dem sich das untere Konnektorelement 13 in dieser Stellung befindet. Diese Ausfädelposition kann so durch eine zugehörige Sensorik detektiert werden. Je nach Anzahl und Positionierung von Empfängerdioden können auch weitere Stellungen des Multikonnektors erkannt werden.

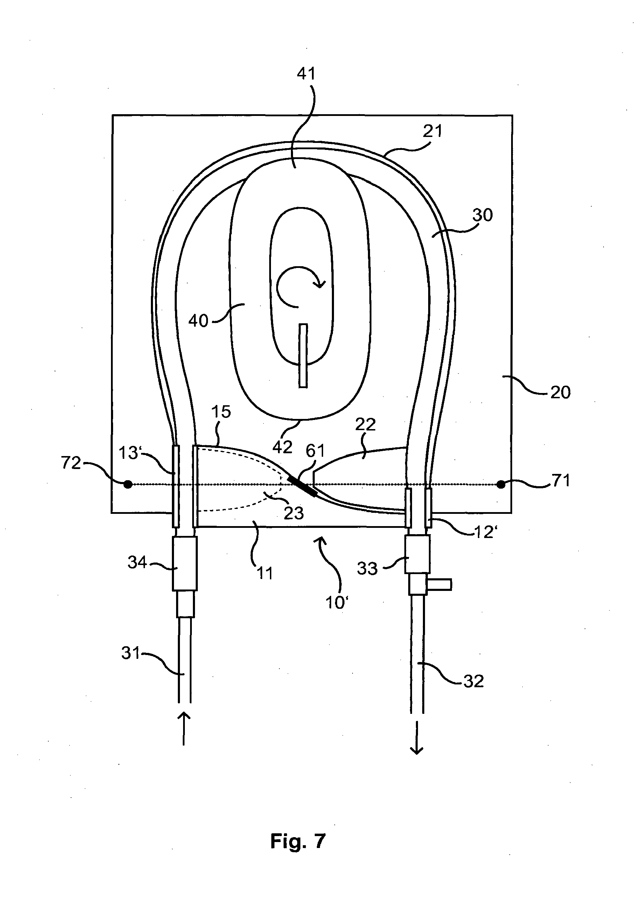

Fig. 7 zeigt eine schematische Darstellung einer Schlauchrollenpumpe mit eingelegtem Schlauchstück 30 und einem Multikonnektor 10' gemäß einer zweiten Ausführungsform, bei welcher der Multikonnektor 10' als Adapter für ein Überleitsystem eingesetzt wird. Der Aufbau dieses Multikonnektors 10' entspricht im Wesentlichen dem Aufbau gemäß der ersten Ausführungsform, bei welcher das Schlauchsegment 30 und die zu- und abführenden Leitungen 31, 32 direkt und fest mit dem Multikonnektor 10 verbunden sind. Das anzuschließende Überleitsystem besteht erneut wenigstens aus einem Schlauchsegment 30 und den zu- und abführenden Leitungen 31 und 32, diese sind jedoch über einfache Verbindungselemente 33 und 34 mit dem Schlauchsegment 30 verbunden.

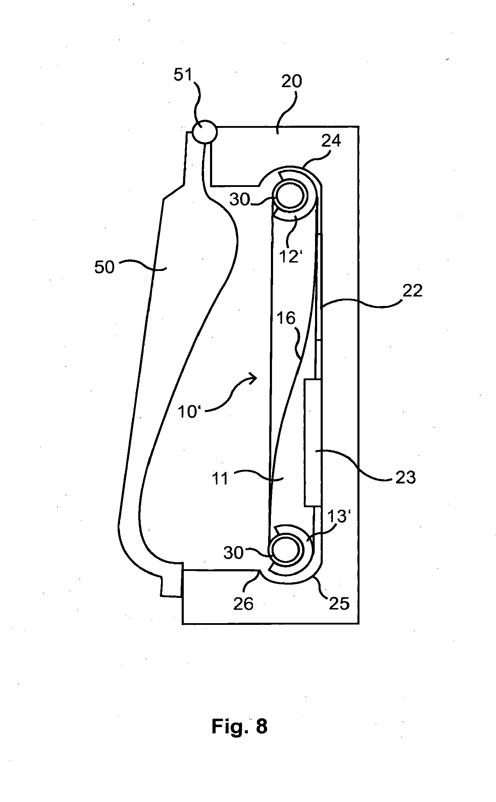

Um auch für ein solches Standard-Überleitsystem die Vorteile des erfindungsgemäßen Multikonnektors 10' und der zugehörigen Multikonnektorerkennung nutzen zu können, sind die Konnektorelemente 12' und 13' des Multikonnektor-Adapters 10' so ausgeformt, dass der Schlauch des Schlauchsegments 30 lösbar an ihnen anbringbar ist. Dazu sind die Konnektorelemente 12' und 13' als Aufnahmen ausgeformt, was beispielsweise durch hohlzylindrische Konnektorelemente 12', 13' erreicht werden kann, die einen Längsschnitt aufweisen, in den das Schlauchsegment 30 eingedrückt werden kann. Diese Einbringung des Schlauchsegments 30 in die Konnektorelemente 12' und 13' ist auch der Seitenansicht der Fig. 8 zu entnehmen.

Nach der Therapie kann das Schlauchsegment 30 wieder aus dem Adapter 10' herausgezogen werden, und ein neues Schlauchsegment 30 kann daran angebracht werden, so dass der Adapter ein wiederverwendbares Bauteil darstellt.

In einer alternativen Ausführungsform des Multikonnektors als Adapter sind die Konnektorelemente so ausgebildet, dass die Verbindungselemente 33 und 34 des anzubringenden Überleitsystems an den Konnektorelementen des Multikonnektors angebracht werden können, um das Überleitsystem mit dem Adapter zu verbinden. Auch hier bieten sich Rastverbindungen an. Auch eine Verbindung über die zu- und abführenden Leitungen oder eine Kombination mehrerer Verbindungsarten wäre möglich.

Die zuvor beschriebenen Ausführungsbeispiele eines Multikonnektors auf vorteilhafte Weise zur Anbringung und Arretierung an einem Pumpengehäuse in wenigstens zwei Stellungen, die dann durch Detektionsmittel der Pumpe erkannt werden können. Allerdings kann der Multikonnektor auch anders ausgeführt sein. Beispielsweise kann er einen anders geformten Grundkörper aufweisen, und die Konnektorelemente müssen nicht parallel zueinander an gegenüber liegenden Seiten eines Grundkörpers angebracht sein. Auch das Prinzip des Drehpunkts an einem Konnektorelement muss nicht verwirklicht sein, und eine Verrastung des Multikonnektors an dem Pumpengehäuse kann ebenfalls auf vielfältige Arten erreicht werden. Somit kann auch die Anbringung und Arretierung am Pumpengehäuse in wenigstens zwei verschiedenen Stellungen unterschiedlich gelöst werden.

Unabhängig von der Ausgestaltung des Multikonnektors im Detail ist er jedoch erfindungsgemäß so ausgebildet, dass er an der Schlauchrollenpumpe in wenigstens zwei Stellungen anbringbar ist, wobei eine erste Stellung eine Therapieposition bzw. Einfädelposition und eine zweite Stellung eine Ausfädelposition für den extrakorporalen Blutkreislauf darstellt. Dazu sind die Geometrie und/oder das Material des Multikonnektors so ausgestaltet, dass wenigstens diese zwei Stellungen des Multikonnektors direkt durch Detektionsmittel an der Schlauchrollenpumpe detektierbar sind.

### Bezugszeichenliste

- 10; 10: Multikonnektor
- 11: Grundkörper
- 12,12',13,13': Konnektorelement, Konnektor
- 14: Zylinderachse
- 15,16: Seitenfläche
- 17: Federelement
- 18: Rastelement
- 19: Zusatzanschluss
- 20: Pumpengehäuse
- 21: Lauffläche
- 22,23: Stützelement
- 24,25: Aufnahme
- 26: Rastkante
- 27: Rastnase
- 30: Pumpensegment, Schlauchsegment
- 31: Leitung, zuführend, arterielles Blut vom Patienten
- 32: Leitung, abführend, venöses Blut vom Patienten
- 33,34: Verbindungselement
- 40: Rotor
- 41,42: Hauptscheitel des Rotors
- 50: Deckel, Pumpendeckel
- 51: Scharnier
- 60: Ausbruch
- 61: Lichtzerstreuende Oberfläche
- 62: Ausbuchtung
- 70: Lichtschranke
- 71,72: Lichtschrankensonde
- 80,80': Lichtleiterweg
- 81: Sendediode
- 82,83: Empfängerdiode
- 90: Medizinisches Gerät zur extrakorporalen Blutbehandlung, Dialysegerät
- 91: Anzeige-/Eingabeeinheit, Touchscreen
- 92: Steuer-/Regelungseinheit
- 93: Dialysator
- 94: Dialysat
- 95: Arterieller Luftfänger
- 96: Venöser Luftfänger

## Patentansprüche

1. Multikonnektor (10; 10') zur werkzeuglosen Anbringung zusammen mit einem Schlauchsegment (30) und zu- und abführenden Leitungen (31, 32) eines extrakorporalen Blutkreislaufs an einer Schlauchrollenpumpe eines medizinischen Geräts, wobei
der Multikonnektor (10; 10') wenigstens einen Grundkörper (11) und zwei Konnektorelemente (12, 13; 12', 13') zur jeweiligen Verbindung des Schlauchsegments (30) mit der zuführenden Leitung (31) und der abführenden Leitung (32) aufweist, und
der Multikonnektor (10; 10') so ausgebildet ist, dass er an der Schlauchrollenpumpe in wenigstens zwei Stellungen anbringbar ist, wobei eine erste Stellung eine Therapieposition und eine zweite Stellung eine Ausfädelposition für den extrakorporalen Blutkreislauf darstellt,
**dadurch gekennzeichnet, dass**
die Geometrie und/oder das Material des Multikonnektors (10; 10') so ausgestaltet sind, dass wenigstens diese zwei Stellungen des Multikonnektors (10; 10') jeweils direkt durch Detektionsmittel an der Schlauchrollenpumpe detektierbar sind, wobei
der Grundkörper (11) des Multikonnektors (10; 10`), wenigstens einen lichtundurchlässigen oder lichtzerstreuenden Abschnitt (61) und angrenzend an diesen Abschnitt (61) oder innerhalb dieses Abschnitts (61) einen lichtdurchlässigen Abschnitt, insbesondere einen Ausbruch (60), aufweist; oder
die Geometrie und das Material des Multikonnektors (10; 10') einen Lichtleiter bilden, durch den Licht entlang eines definierten Weges durch den Multikonnektor (10; 10') leitbar ist.

2. Multikonnektor (10) nach Anspruch 1,
**dadurch gekennzeichnet, dass** die Konnektorelemente (12, 13) fest mit dem Schlauchsegment (30) und den zu- und abführenden Leitungen (31, 32) verbunden sind.

3. Multikonnektor (10') nach Anspruch 1,
**dadurch gekennzeichnet, dass** die Konnektorelemente (12', 13') jeweils Mittel zur lösbaren Verbindung des Schlauchsegments (30) und der zu- und abführenden Leitungen (31, 32) mit dem Multikonnektor (10') aufweisen.

4. Multikonnektor (10; 10') nach einem oder mehreren der Ansprüche 1 bis 3,
**dadurch gekennzeichnet, dass** der Multikonnektor (10; 10') Mittel aufweist, um in den zwei Stellungen an der Schlauchrollenpumpe arretierbar zu sein.

5. Multikonnektor (10; 10') nach einem der Ansprüche 1 bis 4,
**dadurch gekennzeichnet, dass** der lichtundurchlässige oder lichtzerstreuende Abschnitt (61) in einer Stellung des Multikonnektors (10; 10'), insbesondere in der Therapieposition, einen ungehinderten Lichtdurchtritt in eine vorbestimmte Richtung verhindert, und der lichtdurchlässige Abschnitt (60) in einer anderen Stellung des Multikonnektors (10; 10'), insbesondere in der Ausfädelposition, einen ungehinderten Lichtdurchtritt in die vorbestimmte Richtung ermöglicht.

6. Multikonnektor nach einem der Ansprüche 1 bis 5,
**dadurch gekennzeichnet, dass** der Grundkörper (11) außerhalb des lichtundurchlässigen oder lichtstreuenden Bereichs (61) transparent ist.

7. Multikonnektor nach einem der Ansprüche 1 bis 4,
**dadurch gekennzeichnet, dass** der Lichtleiter so ausgebildet ist, dass Licht von einem ersten Konnektorelement (12; 12') durch den Grundkörper (11) zu einem zweiten Konnektorelement (13; 13') leitbar ist.

8. Multikonnektor nach einem oder mehreren der Ansprüche 1 bis 7,
**dadurch gekennzeichnet, dass** am Grundkörper (11) und/oder den Konnektorelementen (12, 13; 12', 13') wenigstens ein Magnet und/oder ein ferromagnetisches Material angebracht ist.

9. Multikonnektor nach einem oder mehreren der Ansprüche 1 bis 8,
**dadurch gekennzeichnet, dass** die Konnektorelemente (12, 13; 12', 13') an zwei gegenüber liegenden Seiten des Grundkörpers (11) angebracht sind, wobei die Außenkontur eines ersten Konnektorelements (12; 12') wenigstens einen zylindrischen Bereich mit einer Zylinderachse (14) aufweist, und von dem ersten Konnektorelement (12; 12') beabstandete Mittel zum Verrasten des Multikonnektors (10; 10') mit einem Pumpengehäuse (20) der Schlauchrollenpumpe vorgesehen sind, wobei die mit diesen Mitteln verrastete Stellung des Multikonnektors (10; 10') die Therapieposition darstellt.

10. Medizinisches Gerät zur extrakorporalen Blutbehandlung, umfassend wenigstens eine Schlauchrollenpumpe mit einem Pumpengehäuse (20), das eine gebogene Lauffläche und einen innerhalb der Lauffläche (21) drehenden Rotor (40) aufweist, wobei ein Schlauchsegment (30) eines extrakorporalen Blutkreislaufs zwischen die Lauffläche (21) und den Rotor (40) einbringbar ist, und dem Schlauchsegment (30) Blut über eine zuführende Leitung (31) zuführbar ist, während Blut von dem Schlauchsegment (30) über eine abführende Leitung (32) abführbar ist,
**dadurch gekennzeichnet, dass**
am Pumpengehäuse (20) ein Multikonnektor (10; 10') nach einem oder mehreren der Ansprüche 1 bis 9 in wenigstens zwei Stellungen werkzeuglos anbringbar ist, wobei eine erste Stellung eine Therapieposition und eine zweite Stellung eine Ausfädelposition darstellt, und
das medizinische Gerät Detektionsmittel zur direkten Detektion wenigstens dieser zwei Stellungen des Multikonnektors (10; 10') aufweist, wobei die Detektion mittels der Geometrie und des Materials des Multikonnektors (10; 10') durchführbar ist.

11. Medizinisches Gerät nach Anspruch 10,
**dadurch gekennzeichnet, dass** am Pumpengehäuse (20) eine Aufnahme (24) vorgesehen ist, in welche ein zylindrischer Bereich des ersten Konnektorelements (12; 12') einbringbar ist, wodurch ein Drehlager gebildet wird, durch das der Multikonnektor (10; 10') um die Zylinderachse (14) des ersten Konnektorelements (12; 12') schwenkbar ist, und dass am Pumpengehäuse (20) Mittel zum Verrasten eines Bereichs des Multikonnektors (10; 10') am Pumpengehäuse (20) vorgesehen sind, der abseits des ersten Konnektorelements (12; 12') liegt.

12. Medizinisches Gerät nach Anspruch 10 oder 11,
**dadurch gekennzeichnet, dass** die Detektionsmittel eine Lichtschranke umfassen, mit der Licht durch den Grundkörper (11) eines Multikonnektors (10; 10') gemäß Anspruch 1 sendbar ist, wobei die Lichtschranke so positioniert ist, dass der lichtdurchlässige Ausbruch (60) des Multikonnektors (10; 10') in einer ersten Stellung des Multikonnektors (10; 10') von der Lichtschranke durchleuchtbar ist, während dieser lichtdurchlässige Ausbruch (60) in einer zweiten Stellung des Multikonnektors (10; 10') nicht von der Lichtschranke durchleuchtbar ist.

13. Medizinisches Gerät nach einem oder mehreren der Ansprüche 10 bis 12,
**dadurch gekennzeichnet, dass** die Detektionsmittel wenigstens eine Sendediode (81) umfassen, mit der Licht in einen Multikonnektor (10; 10') gemäß Anspruch 1 einkoppelbar ist, und wenigstens zwei Empfangsdioden (82; 83) vorgesehen sind, wobei eine erste Empfangsdiode so positioniert ist, dass Licht in einer ersten Stellung des Multikonnektors (10; 10') von der Sendediode (81) zu dieser ersten Empfangsdiode leitbar ist, während eine zweite Empfangsdiode (83) so positioniert ist, dass Licht in einer zweiten Stellung des Multikonnektors (10; 10') von der Sendediode (81) zu dieser zweiten Empfangsdiode (83) leitbar ist.

14. Medizinisches Gerät nach Anspruch 13,
**dadurch gekennzeichnet, dass** die Sendediode (81) im Bereich der Aufnahme (24) angebracht ist, während die erste Empfangsdiode (82) in einem Bereich angebracht ist, in dem das zweite Konnektorelement (13; 13') in der Therapieposition positioniert ist und die zweite Empfangsdiode (83) in einem Bereich angebracht ist, in dem das zweite Konnektorelement (13; 13') in der Ausfädelposition positioniert ist.

15. Medizinisches Gerät nach einem oder mehreren der Ansprüche 10 bis 14, **dadurch gekennzeichnet, dass** die Detektionsmittel wenigstens einen kapazitiv wirkenden Näherungssensor, einen Hall-Sensor und/oder einen induktiv wirkenden Näherungssensor umfassen, mit dem mittels des Materials des Multikonnektors (10; 10') jeweils wenigstens zwei Stellungen des Multikonnektors (10; 10') detektierbar sind.

16. Medizinisches Gerät nach einem oder mehreren der Ansprüche 10 bis 14, **dadurch gekennzeichnet, dass** die Detektionsmittel wenigstens einen Distanzsensor umfassen, mit dem ein veränderlicher Abstand zwischen dem Distanzsensor und dem Multikonnektor (10; 10') in den zwei Stellungen des Multikonnektors (10; 10') messbar ist.

## Claims

1. Multi-connector (10;10') incorporating a hose segment (30) and intake and discharge lines (31, 32) of an extracorporeal blood circuit for mounting on a peristaltic pump of a medical device without tools, wherein
the multi-connector (10; 10') comprises at least a main body (11) and two connector elements (12, 13; 12', 13') for respectively connecting the hose segment (30) to the intake line (31) and to the discharge line (32), and
the multi-connector (10; 10') is configured so that it can be mounted on the peristaltic pump in at least two positions, and a first position constitutes a treatment position and a second position constitutes a thread-out position for the extracorporeal blood circuit,
**characterised in that**
the geometry and/or material of the multi-connector (10; 10') are configured so that at least these two positions of the multi-connector (10; 10') can be respectively directly detected by detection means on the peristaltic pump, and
the main body (11) of the multi-connector (10; 10') has at least one non-transparent or light-scattering portion (61) and adjoining this portion (61) or within this portion a portion permeable to light, in particular a cut-out (60); or
the geometry and the material of the multi-connector (10; 10') act as a light guide by means of which light can be directed through the multi-connector (10; 10') along a defined path.

2. Multi-connector (10) as claimed in claim 1,
**characterised in that** the connector elements (12, 13) are fixedly connected to the hose segment (30) and the intake and discharge lines (31, 32).

3. Multi-connector (10') as claimed in claim 1,
**characterised in that** the connector elements (12', 13') respectively have means for detachably connecting the hose segment (30) and intake and discharge lines (31, 32) to the multi-connector (10').

4. Multi-connector (10; 10') as claimed in one or more of claims 1 to 3,
**characterised in that** the multi-connector (10; 10') has means enabling locking in the two positions on the peristaltic pump.

5. Multi-connector (10; 10') as claimed in one of claims 1 to 4,
**characterised in that** the non-transparent or light-scattering portion (61) prevents unobstructed penetration of light in a predefined direction in one position of the multi-connector (10; 10'), in particular in the treatment position, and the portion (60) permeable to light enables unobstructed penetration of light in the predefined direction in another position of the multi-connector (10; 10'), in particular in the thread-out position.

6. Multi-connector as claimed in one of claims 1 to 5,
**characterised in that** the main body (11) is transparent outside of the non-transparent or light-scattering region (61).

7. Multi-connector as claimed in one of claims 1 to 4,
**characterised in that** the light guide is configured so that light from a first connector element (12; 12') can be directed through the main body (11) to a second connector (13; 13').

8. Multi-connector as claimed in one or more of claims 1 to 7,
**characterised in that** at least one magnet and/or a ferromagnetic material is provided on the main body (11) and/or the connector elements (12, 13; 12', 13').

9. Multi-connector as claimed in one or more of claims 1 to 8,
**characterised in that** the connector elements (12, 13; 12', 13' are mounted on two oppositely lying sides of the main body (11), and the external contour of a first connector element (12; 12') has at least one cylindrical region with a cylinder axis (14), and means at a distance from the first connector element (12; 12') for latching the multi-connector (10; 10') to a pump housing (20) of the peristaltic pump, and the position of the multi-connector (10; 10') latched by these means constitutes the treatment position.

10. Medical device for extracorporeal blood treatment, comprising at least one peristaltic pump with a pump housing (20) having a curved running surface and a rotor (40) rotating inside the running surface (21), and a hose segment (30) of an extracorporeal blood circuit can be inserted between the running surface (21) and the rotor (40), and blood can be delivered to the hose segment (30) via an intake line (31) whilst blood can be discharged from the hose segment (30) via a discharge line (32),
**characterised in that**
a multi-connector (10; 10') as claimed in one or more of claims 1 to 9 can be mounted on the pump housing (20) in at least two positions without tools, and a first position constitutes a treatment position and a second position constitutes a thread-out position, and
the medical device has detection means for directly detecting at least these two positions of the multi-connector (10; 10'), and the detection can be operated on the basis of the geometry and the material of the multi-connector (10; 10').

11. Medical device as claimed in claim 10,
**characterised in that** a holder (24) is provided on the pump housing (20) in which a cylindrical region of the first connector element (12; 12') can be inserted, thereby forming a pivot bearing by means of which the multi-connector (10; 10') can be pivoted about the cylinder axis (14) of the first connector element (12; 12'), and means are provided on the pump housing (20) aside of the first connector element (12; 12') for latching a region of the multi-connector (10; 10') to the pump housing (20).

12. Medical device as claimed in claim 10 or 11,
**characterised in that** the detection means comprise a photoelectric barrier by means of which light can be emitted through the main body (11) of a multi-connector (10; 10') as claimed, in claim 1, and the photoelectric barrier is positioned so that the light-permeable cut-out (60) of the multi-connector (10; 10') can be illuminated by the photoelectric barrier in a first position of the multi-connector (10; 10') whereas in a second position of the multi-connector (10; 10') this light-permeable cut-out (60) cannot be illuminated by the photoelectric barrier.

13. Medical device as claimed in one or more of claims 10 to 12,
**characterised in that** the detection means comprise at least one emitter diode (81) by means of which light can be coupled into a multi-connector (10; 10') as claimed in claim 1, and at least two receiver diodes (82; 83) are provided, and a first receiver diode is positioned so that light from the emitter diode (81) can be directed to this first receiver diode in a first position of the multi-connector (10; 10') whereas a second receiver diode (83) is positioned so that light from the emitter diode (81) can be directed to this second receiver diode (83) in a second position of the multi-connector (10; 10').

14. Medical device as claimed in claim 13,
**characterised in that** the emitter diode (81) is mounted in the region of the holder (24) whereas the first receiver diode (82) is mounted in a region in which the second connector element (13; 13') is positioned in the treatment position and the second receiver diode (83) is mounted in a region in which the second connector element (13, 13') is positioned in the thread-out position.

15. Medical device as claimed in one or more of claims 10 to 14,
**characterised in that** the detection means comprise at least one capacitively acting proximity sensor, a Hall-effect sensor and/or an inductively acting proximity sensor by means of which at least two positions respectively of the multi-connector (10; 10') can be detected by means of the material of the multi-connector (10; 10').

16. Medical device as claimed in one or more of claims 10 to 14,
**characterised in that** the detection means comprise at least one distance sensor, by means of which a variable distance between the distance sensor and the multi-connector (10; 10') can be measured in the two positions of the multi-connector (10; 10').

## Revendications

1. Multiconnecteur (10 ; 10') pour le raccordement sans outil, en même temps que d'un segment de tubulure (30) et de lignes d'amenée et d'évacuation (31, 32) d'une circulation sanguine extracorporelle, à une pompe à galet à tubulure d'un dispositif médical, dans lequel
le multiconnecteur (10 ; 10') comprend au moins un corps de base (11) et deux éléments connecteurs (12, 13; 12', 13'), pour relier le segment de tubulure (30) respectivement à la ligne d'amenée (31) et à la ligne d'évacuation (32), et
le multiconnecteur (10 ; 10') est configuré de façon qu'il puisse être raccordé à la pompe à galet à tubulure dans au moins deux positions, une première position représentant une position de traitement et une deuxième position représentant une position de dégagement pour la circulation sanguine extracorporelle,
**caractérisé en ce que**
la géométrie et/ou le matériau du multiconnecteur (10 ; 10') sont conçus de telle sorte qu'au moins chacune de ces deux positions du multiconnecteur (10 ; 10') puisse être directement détectée par des moyens détecteurs sur la pompe à galet à tubulure,
le corps de base (11) du multiconnecteur (10 ; 10') présente au moins une section (61), opaque à la lumière ou dispersant la lumière, et, adjacente à cette section (61) ou à l'intérieur de cette section (61), une section transparente à la lumière, en particulier un évidement (60) ; ou
la géométrie et le matériau du multiconnecteur (10 ; 10') forment un guide d'onde, par lequel la lumière peut être guidée, le long d'une trajectoire définie, à travers le multiconnecteur (10 ; 10').

2. Multiconnecteur (10) selon la revendication 1, **caractérisé en ce que** les éléments connecteurs (12, 13) sont reliés à demeure avec le segment de tubulure (30) et les lignes d'amenée et d'évacuation (31, 32).

3. Multiconnecteur (10') selon la revendication 1, **caractérisé en ce que** les éléments connecteurs (12', 13') présentent chacun des moyens assurant une liaison amovible respectivement du segment de tubulure (30) et des lignes d'amenée et d'évacuation (31, 32), avec le multiconnecteur (10').

4. Multiconnecteur (10 ; 10') selon l'une ou plusieurs des revendications 1 à 3, **caractérisé en ce que** le multiconnecteur (10 ; 10') comprend des moyens pour pouvoir être bloqué dans les deux positions contre la pompe à galet à tubulure.

5. Multiconnecteur (10 ; 10') selon l'une des revendications 1 à 4, **caractérisé en ce que** la section opaque à la lumière ou dispersant la lumière (61), dans une position du multiconnecteur (10 ; 10'), en particulier dans la position de traitement, empêche un passage libre de la lumière dans une direction prédéterminée, et la section (60) transparente à la lumière, dans une autre position du multiconnecteur (10 ; 10'), en particulier dans la position de dégagement, permet un passage libre de la lumière dans la direction prédéterminée.

6. Multiconnecteur selon l'une des revendications 1 à 5, **caractérisé en ce que** le corps de base (11) est transparent à l'extérieur de la zone opaque à la lumière ou dispersant la lumière (61).

7. Multiconnecteur selon l'une des revendications 1 à 4, **caractérisé en ce que** le guide d'onde est conçu de façon que la lumière provenant du premier élément connecteur (12 ; 12') puisse être guidée, en passant par le corps de base (11), jusqu'à un deuxième élément connecteur (13 ; 13').

8. Multiconnecteur selon l'une ou plusieurs des revendications 1 à 7, **caractérisé en ce qu'**au moins un aimant et/ou un matériau ferromagnétique est fixé au corps de base (11) et/ou aux éléments connecteurs (12, 13 ; 12', 13').

9. Multiconnecteur selon l'une ou plusieurs des revendications 1 à 8, **caractérisé en ce que** les éléments connecteurs (12, 13 ; 12', 13') sont fixés sur deux faces opposées du corps de base (11), le contour extérieur d'un premier élément connecteur (12 ; 12') présentant au moins une zone cylindrique comportant un axe de cylindre (14), et des moyens à distance du premier élément connecteur (12 ; 12') sont prévus pour encliquetage du multiconnecteur (10 ; 10') au un carter (20) de la pompe à galet à tubulure, la position du multiconnecteur (10 ; 10') encliquetée à l'aide de ces moyens représentant la position de traitement.

10. Dispositif médical pour le traitement extracorporel du sang, comprenant au moins une pompe à galet à tubulure comportant un carter de pompe (20), qui présente une surface de roulement incurvée et un rotor (40) tournant à l'intérieur de la surface de roulement (21), un segment de tubulure (30) d'une circulation sanguine extracorporelle pouvant être inséré entre la surface de roulement (21) et le rotor (40), et du sang pouvant être amené au segment de tubulure (30) par l'intermédiaire d'une ligne d'amenée (31), tandis que le sang peut être évacué du segment de tubulure (30) par une ligne d'évacuation (32),
**caractérisé en ce que**
un multiconnecteur (10 ; 10') selon l'une ou plusieurs des revendications 1 à 9 peut être fixé sans outil au carter de pompe (20) dans au moins deux positions, une première position représentant une position de traitement et une deuxième position représentant une position de dégagement, et
le dispositif médical comprend des moyens de détection pour la détection directe d'au moins ces deux positions du multiconnecteur (10 ; 10'), la détection pouvant être mise en oeuvre à l'aide de la géométrie et du matériau du multiconnecteur (10 ; 10').

11. Dispositif médical selon la revendication 10, **caractérisé en ce qu'**un évidement (24) est prévu sur le carter de pompe (20), évidement dans lequel peut être insérée une zone cylindrique du premier élément connecteur (12 ; 12'), ce qui forme un coussinet de pivotement, grâce auquel le multiconnecteur (10 ; 10') peut pivoter autour de l'axe de cylindre (14) du premier élément connecteur (12 ; 12'), et **en ce que** des moyens sont prévus sur le carter de pompe (20) pour encliqueter une zone du multiconnecteur (10 ; 10') au carter de pompe (20), qui est éloigné du premier élément connecteur (12 ; 12').

12. Dispositif médical selon la revendication 10 ou 11, **caractérisé en ce que** les moyens de détection comprennent une barrière lumineuse, à l'aide de laquelle une lumière peut être émise à travers le corps de base (11) d'un multiconnecteur (10 ; 10') selon la revendication 1, la barrière lumineuse étant positionnée de telle sorte que l'évidement transparent à la lumière (60) du multiconnecteur (10 ; 10') puisse, dans une première position du multiconnecteur (10 ; 10'), être traversée par la lumière de la barrière lumineuse, tandis que cet évidement transparent à la lumière (60), dans une deuxième position du multiconnecteur (10 ; 10') ne peut faire passer la lumière provenant de la barrière lumineuse.

13. Dispositif médical selon l'une ou plusieurs des revendications 10 à 12, **caractérisé en ce que** les moyens de détection comprennent au moins une diode émettrice (81), à l'aide de laquelle la lumière peut être injectée dans un multiconnecteur (10 ; 10') selon la revendication 1, et au moins deux diodes réceptrices (82, 83) sont prévues, une première diode réceptrice étant positionnée de telle sorte que la lumière, dans une première position du multiconnecteur (10 ; 10'), puisse être envoyée de la diode émettrice (81) à cette première diode réceptrice, tandis qu'une deuxième diode réceptrice (83) est positionnée de telle sorte que la lumière, dans une deuxième position du multiconnecteur (10 ; 10'), puisse être envoyée par la diode émettrice (81) à cette deuxième diode réceptrice (83).

14. Dispositif médical selon la revendication 13, **caractérisé en ce que** la diode émettrice (81) est disposée dans la zone de l'évidement (24), tandis que la première diode réceptrice (82) est disposée dans une zone dans laquelle le deuxième élément connecteur (13 ; 13') est positionné en position de traitement, et la deuxième diode réceptrice (83) est disposée dans une zone dans laquelle le deuxième élément connecteur (13 ; 13') est positionné en position de dégagement.

15. Dispositif médical selon l'une ou plusieurs des revendications 10 à 14, **caractérisé en ce que** le moyens de détection comprennent au moins un capteur de proximité à effet capacitif, un capteur à effet Hall et/ou un capteur de proximité à effet inductif, à l'aide duquel, par l'intermédiaire du matériau du multiconnecteur (10 ; 10'), il est possible pour chacun d'eux de détecter chacune au moins deux positions du multiconnecteur (10 ; 10').

16. Dispositif médical selon l'une des revendications 10 à 14, **caractérisé en ce que** les moyens de détection comprennent au moins un capteur de distance, à l'aide duquel il est possible de mesurer un écart variable entre le capteur de distance et le multiconnecteur (10 ; 10') dans les deux positions du multiconnecteur (10 ; 10').
